(19) Europäisches Patentamt
European Patent Office
Office européen des brevets

(11) **EP 3 168 605 B1**

(12) **EUROPEAN PATENT SPECIFICATION**

(45) Date of publication and mention
of the grant of the patent:
**04.03.2020 Bulletin 2020/10**

(51) Int Cl.:
*G01N 21/78* (2006.01)          *G01N 1/28* (2006.01)
*G01N 33/52* (2006.01)          *G01N 33/94* (2006.01)

(21) Application number: **15819267.4**

(22) Date of filing: **10.04.2015**

(86) International application number:
**PCT/CN2015/076250**

(87) International publication number:
**WO 2016/004779 (14.01.2016 Gazette 2016/02)**

(54) **BUFFER SYSTEM AND METHOD OF USING THE BUFFER SYSTEM TO MEASURE TOTAL ALKALOID IN TOBACCO OR TOBACCO PRODUCTS THROUGH CONTINUOUS FLOW**

PUFFERSYSTEM UND VERFAHREN ZUR VERWENDUNG DES PUFFERSYSTEMS ZUR MESSUNG DES GESAMTALKALOIDS IN TABAK ODER TABAKPRODUKTEN DURCH KONTINUIERLICHEN FLUSS

SYSTÈME DE TAMPON ET PROCÉDÉ D'UTILISATION DU SYSTÈME DE TAMPON POUR MESURER UN ALCALOÏDE TOTAL DANS DU TABAC OU DES PRODUITS DE TABAC PAR ÉCOULEMENT CONTINU

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **08.07.2014   CN 201410324273**
**08.07.2014   CN 201410322455**

(43) Date of publication of application:
**17.05.2017 Bulletin 2017/20**

(73) Proprietor: **China National Tobacco Quality Supervision & Test Center**
**Zhengzhou, Henan 450001 (CN)**

(72) Inventors:
• **ZHANG, Wei**
**Zhengzhou**
**Henan 450001 (CN)**
• **HU, Qingyuan**
**Zhengzhou**
**Henan 450001 (CN)**
• **LIU, Nan**
**Zhengzhou**
**Henan 450001 (CN)**
• **LUO, Anna**
**Zhengzhou**
**Henan 450001 (CN)**
• **HE, Shengbao**
**Zhengzhou**
**Henan 450001 (CN)**
• **WANG, Yingyuan**
**Zhengzhou**
**Henan 450001 (CN)**
• **FENG, Xiaomin**
**Zhengzhou**
**Henan 450001 (CN)**
• **MA, Yanjun**
**Zhengzhou**
**Henan 450001 (CN)**
• **XING, Jun**
**Zhengzhou**
**Henan 450001 (CN)**
• **HOU, Hongwei**
**Zhengzhou**
**Henan 450001 (CN)**
• **LIU, Yang**
**Zhengzhou**
**Henan 450001 (CN)**
• **WANG, Ying**
**Zhengzhou**
**Henan 450001 (CN)**
• **XIN, Baojun**
**Zhengzhou**
**Henan 450001 (CN)**
• **ZHANG, Hongfei**
**Zhengzhou**
**Henan 450001 (CN)**

**(Cont. next page)**

EP 3 168 605 B1

- **JIANG, Xingyi**
  **Zhengzhou**
  **Henan 450001 (CN)**
- **YANG, Jin**
  **Zhengzhou**
  **Henan 450001 (CN)**

(74) Representative: **Schiweck Weinzierl Koch**
     **Patentanwälte Partnerschaft mbB**
     **Ganghoferstraße 68 B**
     **80339 München (DE)**

(56) References cited:
     WO-A2-2014/093127     CN-A- 1 616 962
     CN-A- 1 635 914       CN-A- 1 795 154
     CN-A- 1 840 669       CN-A- 103 558 349
     CN-A- 104 132 895     CN-A- 104 132 895
     CN-A- 104 132 937     CN-A- 104 132 937

- **"China National Tobacco Quality Supervision
  and Inspection Centre", CHINA NATIONAL
  TOBACCO QUALITY SUPERVISION AND
  INSPECTION CENTRE, 28 February 2013
  (2013-02-28), pages 1-7, XP0008185606,**

- **'Tobacco and Tobacco Products-Determination
  of Total Alkaloid-Continuous Flow (Potassium
  Thiocyanate) Method' TOBACCO
  PROFESSIONAL STANDARD OF THE PEOPLE'S
  REPUBLIC OF CHINA YC/T468-2013 28 February
  2013, page 1, XP008185606**
- **WANG, FANG ET AL.: 'Automatic Analysis on
  Alkaloid in Particulate Matter of Mainstream
  Cigarette Smoke' TOBACCO SCIENCE &
  TECHNOLOGY pages 23 - 25, XP008185503**
- **LI, LING ET AL.: 'Study of Conditions of Tobacco
  Total Alkaloid Determined by Continuous Flow
  Analytical System' JOURNAL OF YUNNAN
  UNIVERSITY ( NATURAL SCIENCES vol. 21,
  pages 114 - 116, XP008185504**

Description

TECHNICAL FIELD

The present invention belongs to the technical field of determining chemical compositions of tobacco, specifically relates to a buffer system and a method for determining total alkaloids in tobacco or tobacco products using the same through a continuous flow method.

BACKGROUND ART

Nicotine is an important composition of tobacco, if a substance does not contain nicotine, the substance cannot be called as tobacco. In the routine analysis of tobacco, generally, the nicotine content is obtained by determining the content of total alkaloids in tobacco. The currently published criteria for determining total alkaloids in tobacco in the tobacco industry contain: GB/T23225-2008 "tobacco and tobacco products, determination of alkaloids, spectrometric method", YC/T160-2002 "tobacco and tobacco products, determination of total alkaloids, continuous flow method" and YC/T468-2013 "tobacco and tobacco products, determination of total alkaloids, continuous flow (potassium thiocyanate) method".

The continuous flow method belongs to an instrumental analysis method. At present, it has been widely used since it is suitable for large volume injection. The determining principle of YC/T160-2002 "tobacco and tobacco products, determination of total alkaloids, continuous flow method" is as follows: generating online cyanogen chloride by using potassium cyanide and chloramine T, breaking the pyridine ring of the nicotine, reacting the resulting substance with p-aminobenzene sulfonic acid, and detecting the resulting product at 460nm using a colorimeter. Although potassium cyanide is stable, it is a highly toxic substance and is ranked in the forefront of toxic chemicals list. There are many problems in the use of potassium cyanide, for example, its sale is supervised rigidly, its purchase period is long for many laboratories, even some laboratories cannot obtain it, and its users need to register in the public security organization. The method requires a suitable acid-base environment, wherein the acid-base environment is controlled by using a buffer solution A and a buffer solution B. In this method, due to weak buffering capacity of the buffer solution B, if the pH value of water in some chemical analytical laboratories is between 5.0 and 6.0, the buffer capacity of the buffer solution B will not satisfy testing needs. The pH value of water used actually in this method is higher than that is required in I grade water in GB6682/T-2008 "water for analytical laboratory use, specification and test methods", in order to meet the requirements for water used in the method, I grade water in the chemical analysis laboratory need to be retreated, this causes that the actual use of this method exist restrictions.

The determining principle of YC/T468-2013 "tobacco and tobacco products, determination of total alkaloids, continuous flow (potassium thiecyanate) method" is as follows: generating online cyanogen chloride by using potassium rhodanide and sodium dichloroisocyanurate, breaking the pyridine ring of the nicotine, reacting the resulting substance with p-aminobenzene sulfonic acid, and detecting the resulting product at 460nm using a colorimeter. Compared with YC/T160-2002, YC/T468-2013 does not use potassium cyanide, the operational safety is improved when determining total alkaloids. Similarly, YC/T 468-2013 also requires a suitable acid-base environment and the acid-base environment also is controlled by using a buffer solution A and a buffer solution B. Even though the buffer system can effectively control the acid-base environment during the reaction, it requires a larger amount of chemicals when formulating the buffer solution A and the buffer solution B, thereby bringing a large inconvenience for this method.

Chinese patent application CN 104 132 895 provides a buffer system suitable for a continuous flow method for measuring total alkaloid in tobacco and tobacco products. The buffer system comprises a buffer solution A and a buffer solution B; wherein the buffer solution A is prepared by the following steps: weighing 60.5 to 83.0 grams of disodium hydrogen phosphate (the preferable amount is 71.6 grams) and 10.1 to 20.2 grams of trisodium phosphate (the preferable amount is 15.2 grams), placing the weighed substances into a beaker, dissolving the mixture with water, then transferring the solution into a volumetric flask, and then filling water into the volumetric flask until the water reaches the scale; and the buffer solution B is prepared by the following steps: weighing 5.5 to 8.0 grams of p-aminobenzene sulfonic acid (the preferable amount is 7.0 grams), 60.5 to 83.0 grams of disodium hydrogen phosphate (the preferable amount is 71.6 grams), 5.0 to 7.0 grams of sodium dihydrogen phosphate (the preferable amount is 6.2 grams), and 10.0 to 12.1 grams of sodium citrate (the preferable amount is 11.8 grams), placing the weighed substances into a beaker, dissolving the mixture with water, transferring the solution into a volumetric flask, and then filling water into the volumetric flask until the water reaches the scale.

Chinese patent application CN 104 132 937 A provides a continuous flow method for measuring total alkaloid in tobacco or tobacco products. In the method, potassium thiocyanate and sodium dichloro isocyanurate are adopted to carry out reactions to generate cyanogen chloride on line. The cyanogen chloride can carry out reactions with total alkaloid (calculated in the form of nicotine) in tobacco or tobacco products to break off the pyridine rings of nicotine, and then further carries out reactions with p-aminobenzene sulfonic acid. The reaction products are measured by a chromometer at 460 nm. The method uses a buffer system to control the pH value of the reaction system in a range of 6.0 to 7.5, and the buffer system is composed of a buffer solution A and a buffer solution B. The buffer solution A is prepared by the following steps: weighing disodium hydrogen phosphate and trisodium phosphate, placing the weighed substances in a beaker, dissolving the substances with water, transferring the solution to a volumetric flask (1L), and adding water into the volumetric flask until the water reaches the scale. The buffer solution B is prepared by the following steps: weighing p-aminobenzene sulfonic acid, disodium hydrogen phosphate, sodium dihydrogen phosphate, and sodium citrate, placing the weighed substances into a beaker, dissolving the substances with water, transferring the solution to a volumetric flask (1L), and then adding water into the volumetric flask until the water reaches the scale.

## SUMMARY OF THE INVENTION

In order to overcome the deficiencies of the prior art, the present invention provides a new buffer system and a continuous flow method for determining total alkaloids in tobacco or tobacco products by using the buffer system. The buffer system is suitable for determining total alkaloids in tobacco or tobacco products by a continuous flow (potassium rhodanide) method. The use of the buffer system of the present invention can both reduce the used amount of chemicals during formulation and control the pH value of reaction solution, which ensures that cyanide chloride can break the pyridine ring of alkaloids, and then react with p-aminobenzene sulfonic acid under the desired acid-base environment, meanwhile, the use of the buffer system of the present invention can both reduce the used amount of chemicals during formulation and keep the testing results consistent with that from the YC/T 468-2013 method.

The present invention is achieved by adopting the following technical solutions:

In one aspect, the present invention provides a buffer system for determining total alkaloids in tobacco and tobacco products by a continuous flow method, comprising a buffer solution A and a buffer solution B;

wherein the buffer solution A is prepared according to the following steps: weighing 60.5 g to 83.0 g, preferably 71.6 g of disodium hydrogen phosphate and 10.1g to 20.2g, preferably 15.2g of trisodium phosphate, placing them into a beaker, dissolving with water, transferring the resulting solution into a volumetric flask of 1 L, and then supplementing water into the volumetric flask to reach the mark;

the buffer solution B is prepared according to the following steps: weighing 5.5 to 8.0 g, preferably 7.0 g of p-aminobenzene sulfonic acid, 60.5g to 83.0 g, preferably 71.6 g of disodium hydrogen phosphate, 5.0g to 7.0 g, preferably 6.2g of sodium dihydrogen phosphate, and 10.0g to 12.1g, preferably 11.8g of sodium citrate, placing them into a beaker, dissolving with water, transferring the resulting solution into a volumetric flask of 1 L, and then supplementing water into the volumetric flask to reach the mark;

the ratio of the amount of the buffer solution A to the amount of the buffer solution B is 3:4.

Disodium hydrogen phosphate, trisodium phosphate, sodium dihydrogen phosphate and sodium citrate may exist in conventional forms, for example. disodium hydrogen phosphate may exist in the form of disodium hydrogen phosphate containing 12 crystalwater, trisodium phosphate may exist in the form of trisodium phosphate containing 12 crystalwater, sodium dihydrogen phosphate may exist in the form of sodium dihydrogen phosphate containing 2 crystalwater, sodium citrate may exist in the form of sodium citrate containing 2 crystalwater.

Also disclosed herein is that, in the above buffer system, the ratio of the amount of the buffer solution A to the amount of the buffer solution B may be 1: 2 to 1: 1 (by volume). According to the invention, in the above buffer system, the ratio of the amount of the buffer solution A to the amount of the buffer solution B is 3: 4 (by volume).

In another aspect, the present invention provides a continuous flow method for determining total alkaloids in tobacco or tobacco products, comprising generating online cyanogen chloride by using potassium rhodanide and sodium dichloroisocyanurate, reacting cyanogen chloride with total alkaloids (by nicotine) in the tobacco or the tobacco products, breaking the pyridine ring of the nicotine, reacting the resulting substance with p-aminobenzene sulfonic acid, and detecting the resulting product at 460nm using a colorimeter, the method comprising:

controlling pH value of the reaction system between 6.0 and 7.5 using a buffer solution A and a buffer solution B;

wherein the buffer solution A is prepared according to the following steps: weighing 60.5 g to 83.0 g, preferably 71.6 g of disodium hydrogen phosphate and 10.1g to 20.2g, preferably 15.2g of trisodium phosphate, placing them into a beaker, dissolving with water, transferring the resulting solution into a volumetric flask of 1 L, and then supplementing water into the volumetric flask to reach the mark;

the buffer solution B is prepared according to the following steps: weighing 5.5 to 8.0 g, preferably 7.0 g of p-aminobenzene sulfonic acid, 60.5g to 83.0 g, preferably 71.6 g of disodium hydrogen phosphate, 5.0g to 7.0 g, preferably 6.2g of sodium dihydrogen phosphate, and 10.0g to 12.1g, preferably 11.8g of sodium citrate, placing them into a beaker, dissolving with water, transferring the resulting solution into a volumetric flask of 1 L, and then supplementing water into the volumetric flask to reach the mark; and

the ratio of the amount of the buffer solution A to the amount of the buffer solution B is 3:4.

Disodium hydrogen phosphate, trisodium phosphate, sodium dihydrogen phosphate and sodium citrate may exist in conventional forms, for example, disodium hydrogen phosphate may exist in the form of disodium hydrogen phosphate containing 12 crystalwater, trisodium phosphate may exist in the form of trisodium phosphate containing 12 crystalwater, sodium dihydrogen phosphate may exist in the form of sodium dihydrogen phosphate containing 2 crystalwater, sodium citrate may exist in the form of sodium citrate containing 2 crystalwater.

Also disclosed herein is that, in the above method, the ratio of the amount of the buffer solution A to the amount of the buffer solution B may be 1: 2 to 1: 1(by volume). According to the invention, in the above method, the ratio of the amount of the buffer solution A to the amount of the buffer solution B is 3: 4 (by volume).

Preferably, the method further comprises: preparing a sample according to the following steps: preparing a tobacco sample according to the principle of YC / T31, detecting the moisture content, extracting the tobacco sample with 5% acetic acid, and then filtering to obtain a filtrate.

Preferably, the method further comprises: preparing a standard solution according to the following steps: weighing nicotine or nicotine salts, dissolving the same with 5% acetic acid, formulating into at least five standard solutions, the nicotine content in the formulated standard solutions ranges from 0.5% to 7%;

Preferably, the method further comprises: preparing a detoxification solution A according to the following steps: weighing 1g of citric acid, and 10g of ferrous sulfate, placing them into a beaker, dissolving with water, transferring the resulting solution into a volumetric flask of 1L, and then supplementing water into the volumetric flask to reach the mark; preparing a detoxification solution B according to the following steps: weighing 10g of sodium carbonate, placing it into a beaker, dissolving with water, transferring the resulting solution into a volumetric flask of 1L, and then supplementing water into the volumetric flask to reach the mark; preparing a potassium rhodanide solution according to the following steps: weighing potassium rhodanide, placing it into a beaker, dissolving with water, transferring the resulting solution into a volumetric flask of 250mL, and then supplementing water into the volumetric flask to reach the mark, the concentration of the solution ranges from 0.09 to 0.21mol/L, preferably 0.12 mol/L; preparing a sodium dichloroisocyanurate solution according to the following steps: weighing sodium dichloroisocyanurate, placing it into a beaker, dissolving with water, transferring the resulting solution into a volumetric flask of 250mL, and then supplementing water into the volumetric flask to reach the mark, the concentration of the solution ranges from 0.02 to 0.06mol/L, preferably 0.04 mol/L;

Preferably, in the above method, the ratio of the concentration of the potassium rhodanide solution to the concentration of the sodium dichloroisocyanurate solution is 2.5: 1 to 3.5: 1, preferably 3: 1.

Preferably, the method further comprises:

detecting by a continuous flow analyzer, then calculating total alkaloids in the sample according to the following formula:

$$\text{total alkaloids\%} = \frac{X \times V}{m \times (1 - W) \times 1000} \times 100$$

in the formula,

X: observed value of the sample(s) in milligrams per milliliter (mg/mL);
V: volume of the added extract solution in milliliters (mL);
m: mass of the sample(s) in grams (g);
W: percentage of moisture in the sample(s), % (mass fraction);

the total alkaloid in the above formula is based on dry basis.

Preferably, the operating parameters of the continuous flow analyzer in the above method are: the ratio of the sampling time to the cleaning time is 1.5, the sampling frequency is 48 cups/hour, the gain is 18-60, and the light intensity is 2.85 volts.

Preferably, the method comprises:

(1) preparation of samples: preparing a tobacco sample according to the principle of YC / T31, detecting the moisture content, extracting the tobacco sample with 5% acetic acid, and then filtering to obtain a filtrate for further use;

(2) preparation of standard solutions: weighing nicotine or nicotine salts, dissolving the same with 5% acetic acid, formulating into at least five standard solutions, the nicotine content in the formulated standard solutions ranges from 0.5% to 7%;

(3) preparation of reagents:
A potassium rhodanide solution was prepared according to the following steps: weighing potassium rhodanide, placing it into a beaker, dissolving with water, transferring the resulting solution into a volumetric flask of 250mL, and then supplementing water into the volumetric flask to reach the mark, the concentration of the solution ranges from 0.09 to 0.21mol/L, preferably 0.12 mol/L.

A sodium dichloroisocyanurate solution was prepared according to the following steps: weighing sodium dichloroisocyanurate, placing it into a beaker, dissolving with water, transferring the resulting solution into a volumetric flask of 250mL, and then supplementing water into the volumetric flask to reach the mark, the concentration of the solution ranges from 0.02 to 0.06mol/L, preferably 0.04 mol/L.

A buffer solution A was prepared according to the following steps: weighing 60.5 g to 83.0 g, preferably 71.6 g of disodium hydrogen phosphate and 10.1g to 20.2g, preferably 15.2g of trisodium phosphate, placing them into a beaker, dissolving with water, transferring the resulting solution into a volumetric flask of 1 L, and then supplementing water into the volumetric flask to reach the mark.

A buffer solution B was prepared according to the following steps: weighing 5.5 to 8.0 g, preferably 7.0 g of p-aminobenzene sulfonic acid, 60.5g to 83.0 g, preferably 71.6 g of disodium hydrogen phosphate, 5.0g to 7.0 g, preferably 6.2g of sodium dihydrogen phosphate, and 10.0g to 12.1g, preferably 11.8g of sodium citrate, placing them into a beaker, dissolving with water, transferring the resulting solution into a volumetric flask of 1 L, and then supplementing water into the volumetric flask to reach the mark.

The disodium hydrogen phosphate, trisodium phosphate, sodium dihydrogen phosphate and sodium citrate may exist in conventional forms, for example, disodium hydrogen phosphate may exist in the form of disodium hydrogen phosphate containing 12 crystalwater, trisodium phosphate may exist in the form of trisodium phosphate containing 12 crystalwater, sodium dihydrogen phosphate may exist in the form of sodium dihydrogen phosphate containing 2 crystalwater, sodium citrate may exist in the form of sodium citrate containing 2 crystalwater.

A detoxification solution A was prepared according to the following steps: weighing 1g of citric acid, and 10g of ferrous sulfate, placing them into a beaker, dissolving with water, transferring the resulting solution into a volumetric flask of 1L, and then supplementing water into the volumetric flask to reach the mark.

A detoxification solution B was prepared according to the following steps: weighing 10g of sodium carbonate, placing it into a beaker, dissolving with water, transferring the resulting solution into a volumetric flask of 1L, and then supplementing water into the volumetric flask to reach the mark.

(4) detecting the filtrate obtained in step (1) and the standard solution obtained in step (2) in the reaction system in step (3) using a continuous flow analyzer;

(5) calculating the total alkaloids in the sample(s) according to the following formula:

$$\text{total alkaloids\%} = \frac{X \times V}{m \times (1 - W) \times 1000} \times 100$$

in the formula,

X: observed value of the sample(s) in milligrams per milliliter (mg/mL);
V: volume of the added extract solution in milliliters (mL);
m: mass of the sample(s) in grams (g);
W: percentage of moisture in the sample(s), % (mass fraction);

the total alkaloid in the above formula is based on dry basis.

Also disclosed herein is that, in the above method, the ratio of the amount of the buffer solution A to the amount of the buffer solution B may be 1: 2 to 1: 1 (by volume). According to the invention, in the above method, the ratio of the amount of the buffer solution A to the amount of the buffer solution B is 3: 4 (by volume), which controls the pH value of the reaction system between 6.0 and 7.5.

Preferably, in the above method, the ratio of the concentration of the potassium rhodanide solution to the concentration of the sodium dichloroisocyanurate solution is 2.5: 1 to 3.5: 1, preferably 3: 1.

Preferably, in the above method, the operating parameters of the continuous flow analyzer in the method are: the ratio of the sampling time to the cleaning time is 1.5, the sampling frequency is 48 cups/hour, the gain is 18-60, and the light intensity is 2.85 volts.

In the above method, the order of preparing the samples, the standard solution and the reagents may vary.

In one specific embodiment, the method of the present invention comprises the following steps:

(1) extracting total alkaloids in tobacco powder sample (which is prepared according to YC/T31) with 5% acetic acid;

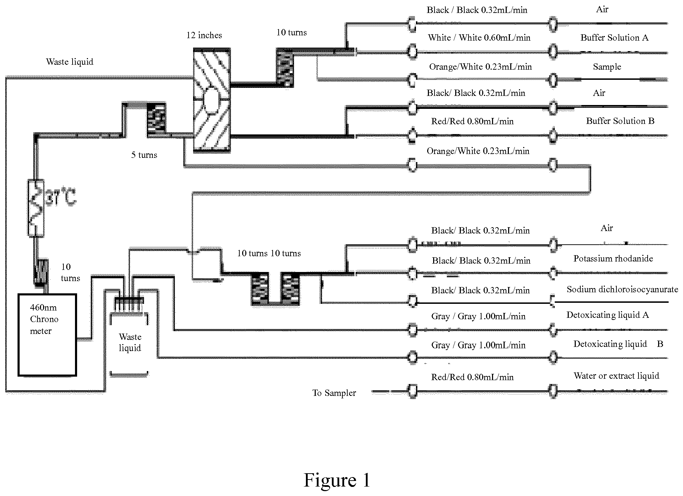

(2) connecting pipeline according to figure 1;

(3) preparing a certain concentration of buffer solution A, buffer solution B, detoxification solution A, detoxification solution B, potassium rhodanide solution and sodium dichloroisocyanurate solution;

(4) preparing a standard solution of total alkaloids with 5% acetic acid;

(5) snapping the tube in the total alkaloids module into the peristaltic pump as shown in figure 1 (the flow rate of the reagents through different pump tubes on the module is different due to different inner diameters of these pump tubes, the proportion of reagents involved in the reaction can be controlled by controlling the flow rate of the reagents; the addition of the air pump tube is to mix uniformly the solution in the pipeline), covering the lid on the pump, and putting the tube containing the reagents into the water; turning on the power, detector, peristaltic pump, autosampler, and computer; moving the switch of the peristaltic pump to "Run"; double-clicking the continuous flow analysis workstation in the computer; clicking "charting" of the workstation, after connecting successfully, the peristaltic pump starts running, and the water is pumped into the pipeline to clean for 20 ~ 30 min; putting the reagents into the corresponding pipeline when the baseline is stable (when the fluctuation of baseline is less than ± 1%), and then continuing running, adjusting the baseline into about 5% in the "charting" window of the workstation during running; writing the running program according to the prompts of "Analysis" from the drop-down menu of the workstation, clicking "Run" of the workstation to run when the baseline is stable; and putting different pipelines back into water to clean for about 20~30min again after the program is over; closing the autosampler, peristaltic pump, and detector successively after finishing cleaning, and taking down the lid on the pump and turning off power.

The above step (1) is as follows: weighing the tobacco powder sample (which is prepared according to the principle of YC / T31), placing it into a 50mL stoppered flask, adding with 5% acetic acid, oscillating for 30min, and filtering by a qualitative filter paper.

The above step (2) is as follows: as shown in figure 1, installing the tube into the peristaltic pump, covering the lid and washing the pipeline with water.

In the above step (3), the buffer solution A is prepared according to the following steps: weighing 71.6 g of disodium hydrogen phosphate and 15.2 g of trisodium phosphate, placing them into a beaker, dissolving with water, transferring the resulting solution into a volumetric flask of 1 L, and then supplementing water into the volumetric flask to reach the mark. Wherein, the disodium hydrogen phosphate, trisodium phosphate may exist in conventional forms, for example, disodium hydrogen phosphate may exist in the form of disodium hydrogen phosphate containing 12 crystalwater, trisodium phosphate may exist in the form of trisodium phosphate containing 12 crystalwater.

In the above step (3), the buffer solution B is prepared according to the following steps: weighing 7.0 g of p-aminobenzene sulfonic acid, 71.6 g of disodium hydrogen phosphate, 6.2 g of sodium dihydrogen phosphate and 11.8 g of sodium citrate, placing them into a beaker, dissolving with water, transferring the resulting solution into a volumetric flask of 1 L, and then supplementing water into the volumetric flask to reach the mark. Wherein, the disodium hydrogen phosphate, sodium dihydrogen phosphate and sodium citrate may exist in conventional forms, for examples, disodium hydrogen phosphate may exist in the form of disodium hydrogen phosphate containing 12 crystalwater, sodium dihydrogen phosphate may exist in the form of sodium dihydrogen phosphate containing 2 crystalwater, sodium citrate may exist in the form of sodium citrate containing 2 crystalwater.

In the above step (3), the detoxification solution A is prepared according to the following steps: weighing 1g of citric acid, and 10g of ferrous sulfate, placing them into a beaker, dissolving with water, transferring the resulting solution into a volumetric flask of 1L, and then supplementing water into the volumetric flask to reach the mark. The main function of the detoxification solution A is to decrease the toxicity of unreacted cyanogen chloride. The detoxification solution A in the step (3) is the same as the detoxification solution A of the YC/T160-2002 (potassium cyanide method) method.

In the above step (3), the detoxification solution B is prepared according to the following steps: weighing 10g of sodium carbonate, placing it into a beaker, dissolving with water, transferring the resulting solution into a volumetric flask of 1L, and then supplementing water into the volumetric flask to reach the mark. The main function of the detoxification solution B is to decrease the toxicity of unreacted cyanogen chloride. The detoxification solution B in the step (3) is the same as the detoxification solution B of the YC/T 160-2002 (potassium cyanide method) method.

In the above step (3), the potassium rhodanide solution is prepared according to the following steps: weighing an appreciate amount of potassium rhodanide, placing it into a beaker, dissolving with water, transferring the resulting solution into a volumetric flask of 250 mL, and then supplementing water into the volumetric flask to reach the mark, the concentration of the solution ranges from 0.09 to 0.21mol / L.

In the above step (3), the sodium dichloroisocyanurate solution is prepared according to the following steps: weighing an appreciate amount of sodium dichloroisocyanurate, placing it into a beaker, dissolving with water, transferring the resulting solution into a volumetric flask of 250 mL, and then supplementing water into the volumetric flask to reach the mark, the concentration of the solution ranges from 0.02 to 0.06mol/L.

In the above step (3), the ratio of the concentration of the potassium rhodanide solution to the concentration of the sodium dichloroisocyanurate solution is about 3: 1.

In the above step (4), the standard solution is prepared according to the following steps: weighing an appreciate amount of nicotine, placing it into a volumetric flask of 250 mL, dissolving with 5% acetic acid, and then supplementing water into the volumetric flask to reach the mark. This solution is a stock solution of nicotine, wherein the nicotine content is about 1.6mg/mL. At least five standard solutions are prepared from the nicotine stock solution. The concentration range of these standard solutions should cover the content of the samples to be detected.

In the above step (5), the operating parameters of the continuous flow analyzer are: the ratio of the sampling time to the cleaning time is 1.5, the sampling frequency is 48 cups/hour, the gain is 18-60, and the light intensity is 2.85 volts.

In the above continuous flow method for determining total alkaloids in tobacco or tobacco products, the pretreatment of samples is the same as that in YC/T468-2013 "tobacco and tobacco products, determination of total alkaloids, continuous flow (potassium thiecyanate) method". The present invention changes the buffer solution A and the buffer solution B and establishes a new buffer system, the new buffer system both reduces the used amount of chemicals when formulating the buffer solution A and the buffer solution B and keeps the testing results consistent with that from the YC/T 468-2013 method.

The present invention has at least the following beneficial effects:
The present invention uses a new buffer solution A, which can control the acid base environment of the reaction before entering the dialyzer, ensure that the pH value of the solution is between 6.0 and 11.0 before entering the dialyzer and will not have effect on the acid base environment controlled by the buffer solution B;

The present invention uses a new buffer solution B, which can control the acid base environment of the reaction, adjust the pH value of the solution between 6.0 and 7.5, ensure that cyanogen chloride breaks the pyridine ring of alkaloids, and then reacts with p-aminobenzene sulfonic acid at the required acid base environment;

The present invention establishes a new buffer system which is suitable for detecting total alkaloids in tobacco or tobacco products by a continuous flow method (potassium rhodanide);

Compared with the YC/T468-2013 method, the use of the buffer system of the present invention can both reduces the used amount of chemicals when formulating the buffer solution A and the buffer solution B and keeps the testing results consistent with that from the YC/T 468-2013 method.

## BRIEF DESCRIPTION OF THE DRAWINGS

Figure 1 is a piping diagram for determining total alkaloids in tobacco and tobacco products using the buffer system of the present invention by a continuous flow method.

## DETAILED DESCRIPTION OF THE PREFERRED EMBODIMENTS

The present invention will be described in details hereinafter in combination of the accompanying the following examples, however, these examples are only intended to illustrate the invention and should not limit the present invention.

**Example 1:** Determination of total alkaloids in flue-cured tobacco

1) Pretreatment of samples

The flue-cured tobacco powder was prepared into a sample according to YC/T31, its water content was detected, 0.25g of the sample was weighed, placed into a 50mL stoppered flask and added with 25mL 5% acetic acid, the stopper was covered, the resulting mixture was oscillated (with a rotate speed of >150rpm) and extracted for 30min. The former 2mL~3mL filtrate was discarded after filtering by a rapid qualitative filter. The remaining filtrate was collected for subsequent analysis.

2) Preparation of reagents

The buffer solution A was prepared according to the following steps: weighing 60.5 g of disodium hydrogen phosphate (containing 12 crystalwater) and 12.2 g of trisodium phosphate(containing 12 crystalwater), placing them into a beaker, dissolving with water, transferring the resulting solution into a volumetric flask of 1 L, and then supplementing water into the volumetric flask to reach the mark.

The buffer solution B was prepared according to the following steps: weighing 5.5 g of p-aminobenzene sulfonic acid, 60.5 g of disodium hydrogen phosphate(containing 12 crystalwater), 5.0 g of sodium dihydrogen phosphate (containing 2 crystalwater) and 10.0 g of sodium citrate(containing 2 crystalwater), placing them into a beaker, dissolving with water, transferring the resulting solution into a volumetric flask of 1 L, and then supplementing water into the volumetric flask to reach the mark.

In the process of determination, the ratio of the amount of the buffer solution A to the amount of the buffer solution B was 3: 4(by volume).

The preparation of detoxification solution A, detoxification solution B, potassium rhodanide solution and sodium dichloroisocyanurate solution are the same with that of the corresponding reagents in YC / T 468-2013 "tobacco and tobacco products, determination of total alkaloids, continuous flow (potassium thiecyanate) method".

The detailed preparation methods were as follows:

The detoxification solution A was prepared according to the following steps: weighing 1g of citric acid, and 10g of ferrous sulfate, placing them into a beaker, dissolving with water, transferring the resulting solution into a volumetric flask of 1000mL, and then supplementing water into the volumetric flask to reach the mark;

The detoxification solution B was prepared according to the following steps: weighing 10g of sodium carbonate, placing it into a beaker, dissolving with water, transferring the resulting solution into a volumetric flask of 1000mL, and then supplementing water into the volumetric flask to reach the mark;

The potassium rhodanide solution was prepared according to the following steps: weighing 2.88g of potassium rhodanide, placing it into a beaker, dissolving with water, transferring the resulting solution into a volumetric flask of 250mL, and then supplementing water into the volumetric flask to reach the mark.

The sodium dichloroisocyanurate solution was prepared according to the following steps: weighing 2.20g of sodium dichloroisocyanurate, placing it into a beaker, dissolving with water, transferring the resulting solution into a volumetric flask of 250mL, and then supplementing water into the volumetric flask to reach the mark. The solution should be prepared when it is used.

3) Preparation of standard solution

An appropriate amount of standard solution of total alkaloids (National Tobacco Quality Supervision and Inspection Center, certified reference material) was weighed with a accuracy of 0.0001g, placed into a volumetric flask of 250mL, dissolved with water, and then supplemented with water to reach the mark of the volumetric flask. The resulting solution is a stock standard solution of nicotine, the nicotine content should be 1.6mg/mL or so. 12.5mL, 10mL, 5mL, 2.5mL and 0.5mL of the stock standard solution of nicotine were pipetted respectively into a volumetric flask of 100mL, and then supplemented with 5% acetic acid into the volumetric flask to reach the mark, the working solutions having the following concentrations were obtained: 1.2105mg/mL, 0.9683mg/mL, 0.4842mg/mL, 0.2421mg/mL, 0.0484mg/mL.

4) The analyzer used in the present invention is AA3 continuous flow Analyzer (AutoAnalyzer III) by German Bran+Luebbe. The piping diagram for detecting total alkaloids in tobacco and tobacco products by a continuous flow method was as shown in figure 1. The tube in the total alkaloids module was snapped into the peristaltic pump as shown in figure 1 (the flow rate of the reagents through different pump tubes on the module is different due to different inner diameters of these pump tubes, the proportion of reagents involved in the reaction can be controlled by controlling the flow rate of the reagents; the addition of the air pump tube is to mix uniformly the solution in the pipeline), the lid was covered on the pump, and the tube containing the reagents was putted into the water; the power, detector, peristaltic pump, autosampler, and computer were turned on; the switch of the peristaltic pump was moved to "Run"; the continuous flow analysis workstation in the computer was double-clicked; "charting" of the workstation was clicked, after connecting successfully, the peristaltic pump started running, and the water was pumped into the pipeline to clean for 20 ~ 30 min; the reagents was putted into the corresponding pipeline when the baseline was stabile (when the fluctuation of baseline was less than ± 1%), and then the running was continued, the baseline was adjusted into about 5% in the "charting" window of the workstation during running; the running program was writtenn according to the prompts of "Analysis" from the drop-down menu of the workstation, "Run" of the workstation was clicked to run when the baseline was stable; and different pipelines was putted back into water to clean for about 20~30min again after the program was over; the autosampler, peristaltic pump, and detector were closed successively after finishing cleaning, and the lid on the pump was taken down and the power was turned off.

5) The first curve was used as the standard curve;

6) Data processing

The peak height of total alkaloids and the corresponding concentration were regressed to obtain a standard curve. On basis of the peak height of the detected sample, the corresponding concentration of the sample was obtained from the standard curve, this value was the observed value.

The content of the total alkaloids in the sample on a dry basis was obtained according to the following formula

$$\text{total alkaloids\%} = \frac{X \times V}{m \times (1 - W) \times 1000} \times 100$$

in the formula,

X: observed value of the sample(s) in milligrams per milliliter (mg/mL);
V: volume of the added extract solution in milliliters (mL) (here the volume is 25mL);
m: mass of the sample(s) in grams (g);
W: percentage of moisture in the sample(s), % (mass fraction);

The content of total alkaloids in the flue-cured tobacco was 0.5 ~ 3% by calculating according to the above formula.

**Example 2:** Determination of total alkaloids in burley tobacco

1) Pretreatment of samples

The burley tobacco powder was prepared into a sample according to YC/T31, its water content was detected, 0.25g of the burley sample was weighed, placed into a 50mL stoppered flask and added with 25mL 5% acetic acid, the stopper was covered, the resulting mixture was oscillated (with a rotate speed of >150rpm) and extracted for 30min. The former 2mL~3mL filtrate was discarded after filtering by a rapid qualitative filter. The remaining filtrate was collected for subsequent analysis.

2) Preparation of reagents

The buffer solution A was prepared according to the following steps: weighing 83.0 g of disodium hydrogen phosphate (containing 12 crystalwater) and 18.4g of trisodium phosphate(containing 12 crystalwater), placing them into a beaker, dissolving with water, transferring the resulting solution into a volumetric flask of 1 L, and then supplementing water into the volumetric flask to reach the mark.

The buffer solution B was prepared according to the following steps: weighing 8.0 g of p-aminobenzene sulfonic acid, 83.0 g of disodium hydrogen phosphate(containing 12 crystalwater), 7.0 g of sodium dihydrogen phosphate (containing 2 crystalwater) and 12.1 g of sodium citrate(containing 2 crystalwater), placing them into a beaker, dissolving with water, transferring the resulting solution into a volumetric flask of 1 L, and then supplementing water into the volumetric flask to reach the mark.

In the process of determination, the ratio of the amount of the buffer solution A to the amount of the buffer solution B was 3: 4 (by volume).

The preparation of detoxification solution A, detoxification solution B, potassium rhodanide solution and sodium dichloroisocyanurate solution are the same with that of the corresponding reagents in YC / T 468-2013 "tobacco and tobacco products, determination of total alkaloids, continuous flow (potassium thiecyanate) method".

The detailed preparation methods were as follows:

The detoxification solution A was prepared according to the following steps: weighing 1g of citric acid, and 10g of ferrous sulfate, placing them into a beaker, dissolving with water, transferring the resulting solution into a volumetric flask of 1000mL, and then supplementing water into the volumetric flask to reach the mark;

The detoxification solution B was prepared according to the following steps: weighing 10g of sodium carbonate, placing it into a beaker, dissolving with water, transferring the resulting solution into a volumetric flask of 1000mL, and then supplementing water into the volumetric flask to reach the mark;

The potassium rhodanide solution was prepared according to the following steps: weighing 2.88g of potassium rhodanide, placing it into a beaker, dissolving with water, transferring the resulting solution into a volumetric flask of 250mL, and then supplementing water into the volumetric flask to reach the mark.

The sodium dichloroisocyanurate solution was prepared according to the following steps: weighing 2.20g of sodium dichloroisocyanurate, placing it into a beaker, dissolving with water, transferring the resulting solution into a volumetric flask of 250mL, and then supplementing water into the volumetric flask to reach the mark. The solution should be prepared when it is used.

3) Preparation of standard solution

An appropriate amount of standard solution of total alkaloids (National Tobacco Quality Supervision and Inspection Center, certified reference material) was weighed with a accuracy of 0.0001g, placed into a volumetric flask of 250mL, dissolved it with water, and then supplemented with water to reach the mark of the volumetric flask. The resulting solution is a stock standard solution of nicotine, the nicotine content should be 1.6mg/mL or so. 12.5mL, 10mL, 5mL, 2.5mL and 0.5mL of the stock standard solution of nicotine were pipetted respectively into a volumetric flask of 100mL, and then supplemented with 5% acetic acid into the volumetric flask to reach the mark, the working solutions having the following concentrations were obtained: 1.2105mg/mL, 0.9683mg/mL, 0.4842mg/mL, 0.2421mg/mL, 0.0484mg/mL.

4) The analyzer used in the present invention is AA3 continuous flow Analyzer (AutoAnalyzer III) by German Bran+Luebbe. The piping diagram for detecting total alkaloids in tobacco and tobacco products through a continuous flow method was as shown in figure 1. The tube in the total alkaloids module was snapped into the peristaltic pump as shown in figure 1 (the flow rate of the reagents through different pump tubes on the module is different due to different inner diameters of these pump tubes, the proportion of reagents involved in the reaction can be controlled by controlling the flow rate of the reagents; the addition of the air pump tube is to mix uniformly the solution in the pipeline), the lid was covered on the pump, and the tube containing the reagents was putted into the water; the power, detector, peristaltic pump, autosampler, and computer were turned on; the switch of the peristaltic pump was moved to "Run"; the continuous flow analysis workstation in the computer was double-clicked; "charting" of the workstation was clicked, after connecting successfully, the peristaltic pump started running, and the water was pumped into the pipeline to clean for 20 ~ 30 min; the reagents was putted into the corresponding pipeline when the baseline was stabile (when the fluctuation of baseline was less than ± 1%), and then the running was continued, the baseline was adjusted into about 5% in the "charting" window of the workstation during running; the running program was written according to the prompts of "Analysis" from the drop-down menu of the workstation, "Run" of the workstation was clicked to run when the baseline was stable; and different pipelines was putted back into water to clean for about 20~30min again after the program was over; the autosampler, peristaltic pump, and detector were closed successively after finishing cleaning, and the lid on the pump was taken down and the power was turned off.

5) The first curve was used as the standard curve;

6) Data processing

The peak height of total alkaloids and the corresponding concentration were regressed to obtain a standard curve. On basis of the peak height of the detected sample, the corresponding concentration of the sample was obtained from the standard curve, this value was the observed value.

The content of the total alkaloids in the sample on a dry basis was obtained according to the following formula

$$\text{total alkaloids\%} = \frac{X \times V}{m \times (1-W) \times 1000} \times 100$$

in the formula,

X: observed value of the sample(s) in milligrams per milliliter (mg/mL);
V: volume of the added extract solution in milliliters (mL) (here the volume is 25mL);
m: mass of the sample(s) in grams (g);
W: percentage of moisture in the sample(s), % (mass fraction);

The content of total alkaloids in burley tobacco was 1 ~ 6% by calculating according to the above formula.

**Example 3:** Determination of total alkaloids in flue-cured tobacco

1) Pretreatment of samples

The flue-cured tobacco powder was prepared into the sample according to YC/T31, its water content was detected, 0.25g of the sample was weighed, placed into a 50mL stoppered flask and added with 25mL 5% acetic acid, the stopper was covered, the resulting mixture was oscillated (with a rotate speed of >150rpm) and extracted for 30min. The former 2mL~3mL filtrate was discarded after filtering by a rapid qualitative filter, the remaining filtrate was collected for subsequent analysis.

2) Preparation of reagents

The buffer solution A was prepared according to the following steps: weighing 71.6 g of disodium hydrogen phosphate (containing 12 crystalwater) and 15.2 g of trisodium phosphate(containing 12 crystalwater), placing them into a beaker, dissolving with water, transferring the resulting solution into a volumetric flask of 1 L, and then supplementing water into the volumetric flask to reach the mark.

The buffer solution B was prepared according to the following steps: weighing 7 g of p-aminobenzene sulfonic acid, 71.6 g of disodium hydrogen phosphate(containing 12 crystalwater), 6.2 g of sodium dihydrogen phosphate (containing 2 crystalwater) and 11.8 g of sodium citrate(containing 2 crystalwater), placing them into a beaker, dissolving with water, transferring the resulting solution into a volumetric flask of 1 L, and then supplementing water into the volumetric flask to reach the mark.

In the process of determination, the ratio of the amount of the buffer solution A to the amount of the buffer solution B was 3: 4 (by volume).

The preparation of detoxification solution A, detoxification solution B, potassium rhodanide solution and sodium dichloroisocyanurate solution are the same with that of the corresponding reagents in YC / T 468-2013 "tobacco and tobacco products, determination of total alkaloids, continuous flow (potassium thiecyanate) method".

The detailed preparation methods were as follows:

The detoxification solution A was prepared according to the following steps: weighing 1g of citric acid, and 10g of ferrous sulfate, placing them into a beaker, dissolving with water, transferring the resulting solution into a volumetric flask of 1000mL, and then supplementing water into the volumetric flask to reach the mark;

The detoxification solution B was prepared according to the following steps: weighing 10g of sodium carbonate, placing it into a beaker, dissolving with water, transferring the resulting solution into a volumetric flask of 1000mL, and then supplementing water into the volumetric flask to reach the mark;

The potassium rhodanide solution was prepared according to the following steps: weighing 2.88g of potassium rhodanide, placing it into a beaker, dissolving with water, transferring the resulting solution into a volumetric flask of 250mL, and then supplementing water into the volumetric flask to reach the mark.

The sodium dichloroisocyanurate solution was prepared according to the following steps: weighing 2.20g of sodium dichloroisocyanurate, placing it into a beaker, dissolving with water, transferring the resulting solution into a volumetric flask of 250mL, and then supplementing water into the volumetric flask to reach the mark. The solution should be prepared when it is used.

3) Preparation of standard solution

An appropriate amount of standard solution of total alkaloids (National Tobacco Quality Supervision and Inspection Center, certified reference material) was weighed with a accuracy of 0.0001g, placed into a volumetric flask of 250mL, dissolved it with water, and then supplemented with water to reach the mark of the volumetric flask. The resulting solution is a stock standard solution of nicotine, the nicotine content should be 1.6mg/mL or so. 12.5mL, 10mL, 5mL, 2.5mL and 0.5mL of the stock standard solution of nicotine were pipetted respectively into a volumetric flask of 100mL, and then supplemented with 5% acetic acid into the volumetric flask to reach the mark, the working solution having the following concentrations were obtained: 1.2105mg/mL, 0.9683mg/mL, 0.4842mg/mL, 0.2421mg/mL, 0.0484mg/mL.

4) The analyzer used in the present invention is AA3 continuous flow Analyzer (AutoAnalyzer III) by German Bran+Luebbe. The piping diagram for detecting total alkaloids in tobacco and tobacco products through a continuous flow method was as shown in figure 1. The tube in the total alkaloids module was snapped into the peristaltic pump as shown in figure 1 (the flow rate of the reagents through different pump tubes on the module is different due to different inner diameters of these pump tubes, the proportion of reagents involved in the reaction can be controlled by controlling the flow rate of the reagents; the addition of the air pump tube is to mix uniformly the solution in the pipeline), the lid was covered on the pump, and the tube containing the reagents was putted into the water; the power, detector, peristaltic pump, autosampler, and computer were turned on; the switch of the peristaltic pump was moved to "Run"; the continuous flow analysis workstation in the computer was double-clicked; "charting" of the workstation was clicked, after connecting successfully, the peristaltic pump started running, and the water was pumped into the pipeline to clean for 20 ~ 30 min; the reagents was putted into the corresponding pipeline when the baseline was stabile (when the fluctuation of baseline was less than ± 1%), and then the running was continued, the baseline was adjusted into about 5% in the "charting" window of the workstation during running; the running program was written according to the prompts of "Analysis" from the drop-down menu of the workstation, "Run" of the workstation was clicked to run when the baseline was stable; and different pipelines was putted back into water to clean for about 20~30min again after the program was over; the autosampler, peristaltic pump, and detector were closed successively after finishing cleaning, and the lid on the pump was taken down and the power was turned off.

5) The first curve was used as the standard curve;

6) Data processing

The peak height of total alkaloids and the corresponding concentration were regressed to obtain a standard curve. On basis of the peak height of the detected sample, the corresponding concentration of the sample was obtained from the standard curve, this value was the observed value.

The content of the total alkaloids in the sample on a dry basis was obtained according to the following formula

$$\text{total alkaloids\%} = \frac{X \times V}{m \times (1-W) \times 1000} \times 100$$

in the formula,

X: observed value of the sample(s) in milligrams per milliliter (mg/mL);
V: volume of the added extract solution in milliliters (mL) (here the volume is 25mL);
m: mass of the sample(s) in grams (g);
W: percentage of moisture in the sample(s), % (mass fraction);

The content of total alkaloids in flue-cured tobacco was 0.5 ~ 3% by calculating according to the above formula.

**Example 4:** Determination of total alkaloids in burley tobacco

1) Pretreatment of samples

The burley tobacco powder was prepared into the sample according to YC/T31, its water content was detected, 0.25g of the burley sample was weighed, placed into a 50mL stoppered flask and added with 25mL 5% acetic acid, the stopper was covered, the resulting mixture was oscillated (with a rotate speed of >150rpm) and extracted for 30min. The former 2mL~3mL filtrate was discarded after filtering by a rapid qualitative filter, the remaining filtrate was collected for subsequent analysis.

2) Preparation of reagents

The buffer solution A was prepared according to the following steps: weighing 71.6 g of disodium hydrogen phosphate (containing 12 crystalwater) and 15.2 g of trisodium phosphate(containing 12 crystalwater), placing them into a beaker, dissolving with water, transferring the resulting solution into a volumetric flask of 1 L, and then supplementing water into the volumetric flask to reach the mark.

The buffer solution B was prepared according to the following steps: weighing 7 g of p-aminobenzene sulfonic acid, 71.6 g of disodium hydrogen phosphate(containing 12 crystalwater), 6.2 g of sodium dihydrogen phosphate (containing 2 crystalwater) and 11.8 g of sodium citrate(containing 2 crystalwater), placing them into a beaker, dissolving with water, transferring the resulting solution into a volumetric flask of 1 L, and then supplementing water into the volumetric flask to reach the mark.

In the process of determination, the ratio of the amount of the buffer solution A to the amount of the buffer solution B was 3: 4 (by volume).

The preparation of detoxification solution A, detoxification solution B, potassium rhodanide solution and sodium dichloroisocyanurate solution are the same with that of the corresponding reagents in YC / T 468-2013 "tobacco and tobacco products, determination of total alkaloids, continuous flow (potassium thiecyanate) method".

The detailed preparation methods were as follows:

The detoxification solution A was prepared according to the following steps: weighing 1g of citric acid, and 10g of ferrous sulfate, placing them into a beaker, dissolving with water, transferring the resulting solution into a volumetric flask of 1000mL, and then supplementing water into the volumetric flask to reach the mark;

The detoxification solution B was prepared according to the following steps: weighing 10g of sodium carbonate, placing it into a beaker, dissolving with water, transferring the resulting solution into a volumetric flask of 1000mL, and then supplementing water into the volumetric flask to reach the mark;

The potassium rhodanide solution was prepared according to the following steps: weighing 2.88g of potassium rhodanide, placing it into a beaker, dissolving with water, transferring the resulting solution into a volumetric flask of 250mL, and then supplementing water into the volumetric flask to reach the mark.

The sodium dichloroisocyanurate solution was prepared according to the following steps: weighing 2.20g of sodium dichloroisocyanurate, placing it into a beaker, dissolving with water, transferring the resulting solution into a volumetric flask of 250mL, and then supplementing water into the volumetric flask to reach the mark. The solution should be prepared when it is used.

3) Preparation of standard solution

An appropriate amount of standard solution of total alkaloids (National Tobacco Quality Supervision and Inspection Center, certified reference material) was weighed with a accuracy of 0.0001g, placed into a volumetric flask of 250mL, dissolved it with water, and then supplemented with water to reach the mark of the volumetric flask. The resulting solution is a stock standard solution of nicotine, the nicotine content should be 1.6mg/mL or so. 12.5mL, 10mL, 5mL, 2.5mL and 0.5mL of the stock standard solution of nicotine were pipetted respectively into a volumetric flask of 100mL, and then supplemented with 5% acetic acid into the volumetric flask to reach the mark, the working solution having the following concentrations were obtained: 1.2105mg/mL, 0.9683mg/mL, 0.4842mg/mL, 0.2421mg/mL, 0.0484mg/mL.

4) The analyzer used in the present invention is AA3 continuous flow Analyzer (AutoAnalyzer III) by German Bran+Luebbe. The piping diagram for detecting total alkaloids in tobacco and tobacco products through a continuous flow method was as shown in figure 1. The tube in the total alkaloids module was snapped into the peristaltic pump as shown in figure 1 (the flow rate of the reagents through different pump tubes on the module is different due to different inner diameters of these pump tubes, the proportion of reagents involved in the reaction can be controlled by controlling the flow rate of the reagents; the addition of the air pump tube is to mix uniformly the solution in the pipeline), the lid was covered on the pump, and the tube containing the reagents was putted into the water; the power, detector, peristaltic pump, autosampler, and computer were turned on; the switch of the peristaltic pump was moved to "Run"; the continuous flow analysis workstation in the computer was double-clicked; "charting" of the workstation was clicked, after connecting successfully, the peristaltic pump started running, and the water was pumped into the pipeline to clean for 20 ~ 30 min; the reagents was putted into the corresponding pipeline when the baseline was stabile (when the fluctuation of baseline was less than ± 1%), and then the running was continued, the baseline was adjusted into about 5% in the "charting" window of the workstation during running; the running program was written according to the prompts of "Analysis" from the drop-down menu of the workstation, "Run" of the workstation was clicked to run when the baseline was stable; and different pipelines was putted back into water to clean for about 20~30min again after the program was over; the autosampler, peristaltic pump, and detector were closed successively after finishing cleaning, and the lid on the pump was taken down and the power was turned off.

5) The first curve was used as the standard curve;

6) Data processing

The peak height of total alkaloids and the corresponding concentration were regressed to obtain a standard curve. On basis of the peak height of the detected sample, the corresponding concentration of the sample was obtained from the standard curve, this value was the observed value.

The content of the total alkaloids in the sample on a dry basis was obtained according to the following formula

$$\text{total alkaloids\%} = \frac{X \times V}{m \times (1-W) \times 1000} \times 100$$

in the formula,

X: observed value of the sample(s) in milligrams per milliliter (mg/mL);
V: volume of the added extract solution in milliliters (mL) (here the volume is 25mL);
m: mass of the sample(s) in grams (g);
W: percentage of moisture in the sample(s), % (mass fraction);

The content of total alkaloids in burley tobacco was 1 ~ 5% by calculating according to the above formula.

**Example 5:** Determination of total alkaloids in cigarette tobacco

1) Pretreatment of samples

The cigarette tobacco cut was prepared into the sample according to YC/T31, its water content was detected, 0.25g of the sample was weighed, placed into a 50mL stoppered flask and added with 25mL 5% acetic acid, the stopper was covered, the resulting mixture was oscillated (with a rotate speed of >150rpm) and extracted for 30min. The former 2mL~3mL filtrate was discarded after filtering by a rapid qualitative filter, the remaining filtrate was collected for subsequent analysis.

2) Preparation of reagents

The buffer solution A was prepared according to the following steps: weighing 71.6 g of disodium hydrogen phosphate (containing 12 crystalwater) and 15.2 g of trisodium phosphate(containing 12 crystalwater), placing them into a beaker, dissolving with water, transferring the resulting solution into a volumetric flask of 1 L, and then supplementing water into the volumetric flask to reach the mark.
The buffer solution B was prepared according to the following steps: weighing 7 g of p-aminobenzene sulfonic acid, 71.6 g of disodium hydrogen phosphate(containing 12 crystalwater), 6.2 g of sodium dihydrogen phosphate (containing 2 crystalwater) and 11.8 g of sodium citrate(containing 2 crystalwater), placing them into a beaker, dissolving with water, transferring the resulting solution into a volumetric flask of 1 L, and then supplementing water into the volumetric flask to reach the mark.
In the process of determination, the ratio of the amount of the buffer solution A to the amount of the buffer solution B was 3: 4 (by volume).
The preparation of detoxification solution A, detoxification solution B, potassium rhodanide solution and sodium dichloroisocyanurate solution are the same with that of the corresponding reagents in YC / T 468-2013 "tobacco and tobacco products, determination of total alkaloids, continuous flow (potassium thiecyanate) method".
The detailed preparation methods were as follows:
The detoxification solution A was prepared according to the following steps: weighing 1g of citric acid, and 10g of ferrous sulfate, placing them into a beaker, dissolving with water, transferring the resulting solution into a volumetric flask of 1000mL, and then supplementing water into the volumetric flask to reach the mark;
The detoxification solution B was prepared according to the following steps: weighing 10g of sodium carbonate, placing it into a beaker, dissolving with water, transferring the resulting solution into a volumetric flask of 1000mL, and then supplementing water into the volumetric flask to reach the mark;
The potassium rhodanide solution was prepared according to the following steps: weighing 2.88g of potassium rhodanide, placing it into a beaker, dissolving with water, transferring the resulting solution into a volumetric flask of 250mL, and then supplementing water into the volumetric flask to reach the mark.
The sodium dichloroisocyanurate solution was prepared according to the following steps: weighing 2.20g of sodium dichloroisocyanurate, placing it into a beaker, dissolving with water, transferring the resulting solution into a volumetric flask of 250mL, and then supplementing water into the volumetric flask to reach the mark. The solution should be prepared when it is used.

3) Preparation of standard solution

An appropriate amount of standard solution of total alkaloids (National Tobacco Quality Supervision and Inspection Center, certified reference material) was weighed with a accuracy of 0.0001g, placed into a volumetric flask of 250mL, dissolved it with water, and then supplemented with water to reach the mark of the volumetric flask. The resulting solution is a stock standard solution of nicotine, the nicotine content should be 1.6mg/mL or so. 12.5mL, 10mL, 5mL, 2.5mL and 0.5mL of the stock standard solution of nicotine were pipetted respectively into a volumetric flask of 100mL, and then supplemented with 5% acetic acid into the volumetric flask to reach the mark, the working solution having the following concentrations were obtained: 1.2105mg/mL, 0.9683mg/mL, 0.4842mg/mL, 0.2421mg/mL, 0.0484mg/mL.

4) The analyzer used in the present invention is AA3 continuous flow Analyzer (AutoAnalyzer III) by German Bran+Luebbe. The piping diagram for detecting total alkaloids in tobacco and tobacco products through a continuous flow method was as shown in figure 1. The tube in the total alkaloids module was snapped into the peristaltic pump as shown in figure 1 (the flow rate of the reagents through different pump tubes on the module is different due to different inner diameters of these pump tubes, the proportion of reagents involved in the reaction can be controlled by controlling the flow rate of the reagents; the addition of the air pump tube is to mix uniformly the solution in the pipeline), the lid was covered on the pump, and the tube containing the reagents was putted into the water; the power, detector, peristaltic pump, autosampler, and computer were turned on; the switch of the peristaltic pump was moved to "Run"; the continuous flow analysis workstation in the computer was double-clicked; "charting" of the workstation was clicked, after connecting successfully, the peristaltic pump started running, and the water was pumped into the pipeline to clean for 20 ~ 30 min; the reagents was putted into the corresponding pipeline when the baseline was stabile (when the fluctuation of baseline was less than ± 1%), and then the running was continued, the baseline was adjusted into about 5% in the "charting" window of the workstation during running; the running program was written according to the prompts of "Analysis" from the drop-down menu of the workstation, "Run" of the workstation was clicked to run when the baseline was stable; and different pipelines was putted back into water to clean for about 20~30min again after the program was over; the autosampler, peristaltic pump, and detector were closed successively after finishing cleaning, and the lid on the pump was taken down and the power was turned off.

5) The first curve was used as the standard curve;

6) Data processing

The peak height of total alkaloids and the corresponding concentration were regressed to obtain a standard curve. On basis of the peak height of the detected sample, the corresponding concentration of the sample was obtained from the standard curve, this value was the observed value.

The content of the total alkaloids in the sample on a dry basis was obtained according to the following formula

$$\text{total alkaloids\%} = \frac{X \times V}{m \times (1 - W) \times 1000} \times 100$$

in the formula,

X: observed value of the sample(s) in milligrams per milliliter (mg/mL);
V: volume of the added extract solution in milliliters (mL) (here the volume is 25mL);
m: mass of the sample(s) in grams (g);
W: percentage of moisture in the sample(s), % (mass fraction);

The content of total alkaloids in cigarette tobacco was 0.5 ~ 4% by calculating according to the above formula.

**Example 6:** Comparative study of the detection results from the continuous flow method using the buffer system of the present invention and that using other buffer systems

Unless otherwise specified, the reagents and methods used by the present example are consistent with that in YC/T 468-2013.

Five kinds of samples, i.e., flue-cured tobacco, burley tobacco, aromatic tobacco, virginian-type cigarette and blended-type cigarette were determined by using the buffer systems used in example 1, example 2 and examples 3-5. The determination results were shown in tables 1 to 3. It can be seen from tables 1 to 3: the results for the same sample from the continuous flow method by using the buffer systems of the present invention (which were used in example 1, example 2 and examples 3-5) and the continuous flow method by using the buffer systems of YC / T 468-2013 were used to perform a paired t-test ($\alpha$ = 0.05), the results from the continuous flow method of the present invention were $P_{Example1}$= 0.85> 0.05, $P_{Example\ 2}$ = 0.92> 0.05, $P_{Example\ 3-5}$ = 0.35 > 0.05, this shows that there is no significant difference between two methods, that's to say, the results from the continuous flow method using the buffer system of the present invention are consistent with that from the YC / T 468-2013 method; among the buffer systems used in example 1, example 2 and examples 3-5, the difference between the buffer system used in examples 3-5 and that used in YC 3-5 / T 468-2013 standard method is minimum, therefore, the buffer system used in examples 3-5 is chosen as the most preferred system.

Table 1 Comparison of the results from the continuous flow method using the buffer system of the present invention (used in example 1) and the YC/T 468-2013 method

| Continuous flow method using the buffer system of the present invention (used in example 1) | | YC/T 468-2013 | Differences between the two results |
|---|---|---|---|
| Numbers of Samples | mean % | mean % | Difference % |
| Flue-cured tobacco | 1.82 | 1.79 | 0.03 |
| Burley tobacco | 4.50 | 4.57 | 0.07 |
| Aromatic tobacco | 0.99 | 1.02 | 0.03 |
| Virginian-type cigarette | 1.71 | 1.69 | 0.02 |
| Blended-type cigarette | 2.11 | 2.08 | 0.03 |

Table 2 Comparison of the results from the continuous flow method using the buffer system of the present invention (used in example 2) and the YC/T 468-2013 method

| Continuous flow method using the buffer system of the present invention(used in example 2) | | YC/T 468-2013 | Differences between the two results |
|---|---|---|---|
| Numbers of samples | mean % | mean % | Difference % |
| Flue-cured tobacco | 1.76 | 1.79 | 0.03 |
| Burley tobacco | 4.51 | 4.57 | 0.06 |
| Aromatic tobacco | 1.05 | 1.02 | 0.03 |
| Virginian-type cigarette | 1.72 | 1.69 | 0.03 |
| Blended-type cigarette | 2.12 | 2.08 | 0.04 |

Table 3 Comparison of the results from the continuous flow method using the buffer system of the present invention (used in examples 3-5) and the YC/T 468-2013 method

| Continuous flow method using the buffer system of the present invention(used in examples 3-5) | | YC/T 468-2013 | Differences between the two results |
|---|---|---|---|
| Numbers of samples | mean % | mean % | Difference % |
| Flue-cured tobacco | 1.80 | 1.79 | 0.01 |
| Burley tobacco | 4.53 | 4.57 | 0.04 |
| Aromatic tobacco | 1.01 | 1.02 | 0.01 |
| Virginian-type cigarette | 1.70 | 1.69 | 0.01 |
| Blended-type cigarette | 2.06 | 2.08 | 0.02 |

In addition, the present inventors also (except for the buffer systems, the remaining reagents are the same as that in examples 3-5) determined the five kinds of samples, i.e., flue-cured tobacco, burley tobacco, aromatic tobacco, virginian-type cigarette and blended-type cigarette by using other buffer systems, the determination results were shown in tables 4 to 9. It can be seen from tables 4 ∼ 9: the absolute differences of results from the method using other buffer systems relative to the YC/T 468-2013 method were all larger. The results for the same sample from the continuous flow method by using the buffer systems of the present invention (which was used in examples 3-5, contains buffer A and buffer B, the ratio between buffer A and buffer B was the same as that in examples 3-5), the continuous flow method by using other buffer system and the YC / T 468-2013 method were used to perform a paired t-test ($\alpha = 0.05$), the results were shown in table 10, it can be seen from table 10 that the results from the continuous flow method by using the buffer system of the present invention was $P_{Example\ 3-5} = 0.35 > 0.05$, this shows that there is no significant difference between the continuous flow method by using the buffer system of the present invention and the YC / T 468-2013 method, that's to say, the results from the continuous flow method using the buffer system of the present invention are consistent with that from the YC / T 468-2013 method. The results from the continuous flow method by using other buffer systems was P <0.05, this shows that there is significant difference between the continuous flow method by using other buffer systems and the YC / T 468-2013 method, that's to say, the results from the continuous flow method using other buffer systems are inconsistent with that from the YC / T 468-2013 method.

Table 4 Comparison of the results from the continuous flow method using the disodium hydrogen phosphate and borax system and the YC/T 468-2013 method

| Disodium hydrogen phosphate and borax system * | | YC/T 468-2013 | Differences between the two results |
|---|---|---|---|
| Numbers of samples | mean % | mean % | Difference % |
| Flue-cured tobacco | 1.54 | 1.79 | 0.25 |
| Burley tobacco | 4.00 | 4.57 | 0.57 |
| Aromatic tobacco | 0.86 | 1.02 | 0.16 |
| Virginian-type cigarette | 1.46 | 1.69 | 0.23 |
| Blended-type cigarette | 1.87 | 2.08 | 0.21 |

* The preparation of the disodium hydrogen phosphate and borax system are as follows: The buffer solution A was prepared according to the following steps: weighing an appropriate amount of disodium hydrogen phosphate and borax, placing them into a beaker, dissolving with water, transferring the resulting solution into a volumetric flask of 1 L, and then supplementing water into the volumetric flask to reach the mark. The pH value of the solution is between 6.0 and 11.0. The buffer solution B was prepared according to the following steps: weighing 7 g of p-aminobenzene sulfonic acid, an appropriate amount of disodium hydrogen phosphate and borax, placing them into a beaker, dissolving with water, transferring the resulting solution into a volumetric flask of 1 L, and then supplementing water into the volumetric flask to reach the mark. The pH value of the solution is between 6.0 and 7.5.

Table 5 Comparison of the results from the continuous flow method using the sodium dihydrogen phosphate and sodium hydroxide system and the YC/T 468-2013 method

| Sodium dihydrogen phosphate and sodium hydroxide system* | | YC/T 468-2013 | Differences between the two results |
|---|---|---|---|
| Numbers of samples | mean % | mean % | Difference % |
| Flue-cured tobacco | 1.63 | 1.79 | 0.16 |
| Burley tobacco | 4.22 | 4.57 | 0.35 |
| Aromatic tobacco | 0.92 | 1.02 | 0.1 |
| Virginian-type cigarette | 1.55 | 1.69 | 0.14 |
| Blended-type cigarette | 1.95 | 2.08 | 0.13 |

* The preparation method of sodium dihydrogen phosphate and sodium hydroxide system are as follows: The buffer solution A was prepared according to the following steps: weighing an appropriate amount of sodium dihydrogen phosphate and sodium hydroxide, placing them into a beaker, dissolving with water, transferring the resulting solution into a volumetric flask of 1 L, and then supplementing water into the volumetric flask to reach the mark. The pH value of the solution is between 6.0 and 11.0. The buffer solution B was prepared according to the following steps: weighing 7 g of p-aminobenzene sulfonic acid, an appropriate amount of sodium dihydrogen phosphate and sodium hydroxide, placing them into a beaker, dissolving with water, transferring the resulting solution into a volumetric flask of 1 L, and then supplementing water into the volumetric flask to reach the mark. The pH value of the solution is between 6.0 and 7.5.

Table 6 Comparison of the results from the continuous flow method using the sodium phosphate and citric acid system and the YC/T 468-2013 method

| Sodium phosphate and citric acid system* | | YC/T 468-2013 | Differences between the two results |
|---|---|---|---|
| Numbers of samples | mean % | mean % | Difference % |
| Flue-cured tobacco | 1.57 | 1.79 | 0.22 |
| Burley tobacco | 3.91 | 4.57 | 0.66 |
| Aromatic tobacco | 0.89 | 1.02 | 0.13 |
| Virginian-type cigarette | 1.31 | 1.69 | 0.38 |
| Blended-type cigarette | 1.82 | 2.08 | 0.26 |
| * The preparation method of the sodium phosphate and citric acid system are as follows: The buffer solution A was prepared according to the following steps: weighing an appropriate amount of sodium phosphate and citric acid, placing them into a beaker, dissolving with water, transferring the resulting solution into a volumetric flask of 1 L, and then supplementing water into the volumetric flask to reach the mark. The pH value of the solution is between 6.0 and 11.0. The buffer solution B was prepared according to the following steps: weighing 7 g of p-aminobenzene sulfonic acid, an appropriate amount of sodium phosphate and citric acid, placing them into a beaker, dissolving with water, transferring the resulting solution into a volumetric flask of 1 L, and then supplementing water into the volumetric flask to reach the mark. The pH value of the solution is between 6.0 and 7.5. | | | |

Table 7 Comparison of the results from the continuous flow method using the sodium citrate and citric acid system and the YC/T 468-2013 method

| Sodium citrate and citric acid system* | | YC/T 468-2013 | Differences between the two results |
|---|---|---|---|
| Numbers of samples | mean % | mean % | Difference % |
| Flue-cured tobacco | 1.47 | 1.79 | 0.32 |
| Burley tobacco | 3.88 | 4.57 | 0.69 |
| Aromatic tobacco | 0.76 | 1.02 | 0.26 |
| Virginian-type cigarette | 1.36 | 1.69 | 0.33 |
| Blended-type cigarette | 1.97 | 2.08 | 0.11 |
| * The preparation method of the sodium citrate and citric acid system are as follows: The buffer solution A was prepared according to the following steps: weighing an appropriate amount of sodium citrate and citric acid, placing them into a beaker, dissolving with water, transferring the resulting solution into a volumetric flask of 1 L, and then supplementing water into the volumetric flask to reach the mark. The pH value of the solution is between 6.0 and 11.0. The buffer solution B was prepared according to the following steps: weighing 7 g of p-aminobenzene sulfonic acid, an appropriate amount of sodium citrate and citric acid, placing them into a beaker, dissolving with water, transferring the resulting solution into a volumetric flask of 1 L, and then supplementing water into the volumetric flask to reach the mark. The pH value of the solution is between 6.0 and 7.5. | | | |

Table 8 Comparison of the results from the continuous flow method using the barbital sodium and hydrochloric acid system and the YC/T 468-2013 method

| Barbital sodium and hydrochloric acid system* | | YC/T 468-2013 | Differences between the two results |
|---|---|---|---|
| Numbers of samples | mean % | mean % | Difference % |
| Flue-cured tobacco | 1.65 | 1.79 | 0.14 |
| Burley tobacco | 4.31 | 4.57 | 0.26 |

(continued)

| Barbital sodium and hydrochloric acid system* | | YC/T 468-2013 | Differences between the two results |
|---|---|---|---|
| Numbers of samples | mean % | mean % | Difference % |
| Aromatic tobacco | 0.95 | 1.02 | 0.07 |
| Virginian-type cigarette | 1.56 | 1.69 | 0.13 |
| Blended-type cigarette | 1.91 | 2.08 | 0.17 |
| * The preparation of the barbital sodium and hydrochloric acid system are as follows: The buffer solution A was prepared according to the following steps: weighing an appropriate amount of barbital sodium and hydrochloric acid, placing them into a beaker, dissolving with water, transferring the resulting solution into a volumetric flask of 1 L, and then supplementing water into the volumetric flask to reach the mark. The pH value of the solution is between 6.0 and 11.0. The buffer solution B was prepared according to the following steps: weighing 7 g of p-aminobenzene sulfonic acid, an appropriate amount of barbital sodium and hydrochloric acid, placing them into a beaker, dissolving with water, transferring the resulting solution into a volumetric flask of 1 L, and then supplementing water into the volumetric flask to reach the mark. The pH value of the solution is between 6.0 and 7.5. | | | |

Table 9 Comparison of the results from the continuous flow method using the tris(hydroxymethyl)aminomethane and hydrochloric acid system and the YC/T 468-2013 method

| Tris(hydroxymethyl)aminomethane and hydrochloric acid system* | | YC/T 468-2013 | Differences between the two results |
|---|---|---|---|
| Numbers of samples | mean % | mean % | Difference % |
| Flue-cured tobacco | 1.61 | 1.79 | 0.18 |
| Burley tobacco | 4.25 | 4.57 | 0.32 |
| Aromatic tobacco | 0.90 | 1.02 | 0.12 |
| Virginian-type cigarette | 1.52 | 1.69 | 0.17 |
| Blended type cigarette | 1.84 | 2.08 | 0.24 |
| * The preparation of tris(hydroxymethyl)aminomethane and hydrochloric acid system are as follows: The buffer solution A was prepared according to the following steps: weighing an appropriate amount of tris(hydroxymethyl) aminomethane and hydrochloric acid, placing them into a beaker, dissolving with water, transferring the resulting solution into a volumetric flask of 1 L, and then supplementing water into the volumetric flask to reach the mark. The pH value of the solution is between 6.0 and 11.0. The buffer solution B was prepared according to the following steps: weighing 7 g of p-aminobenzene sulfonic acid, an appropriate amount of tris(hydroxymethyl) aminomethane and hydrochloric acid, placing them into a beaker, dissolving with water, transferring the resulting solution into a volumetric flask of 1 L, and then supplementing water into the volumetric flask to reach the mark. The pH value of the solution is between 6.0 and 7.5. | | | |

Table 10 Paired t-test results from different methods by using different buffer systems and the YC / T 468-2013 method

| Names of buffer systems | P value of t test |
|---|---|
| The buffer system of the present invention (which was used in examples 3-5, contains buffer A and buffer B, the ratio of the amount of the buffer solution A to the amount of the buffer solution B was the same as that in examples 3-5) | 0.35 |
| Disodium hydrogen phosphate and borax system | 0.02 |
| Sodium dihydrogen phosphate and sodium hydroxide system | 0.02 |
| Sodium phosphate and citric acid system | 0.02 |
| Sodium citrate and citric acid system | 0.02 |

(continued)

| Names of buffer systems | P value of t test |
|---|---|
| Barbital sodium and hydrochloric acid system | 0.01 |
| Tris(hydroxymethyl)aminomethane and hydrochloric acid system | 0.003 |

**Claims**

1. A buffer system for determining total alkaloids in tobacco or tobacco products by a continuous flow method, comprising a buffer solution A and a buffer solution B;
   wherein the buffer solution A is prepared according to the following steps: weighing 60.5 g to 83.0 g, preferably 71.6 g of disodium hydrogen phosphate and 10.1g to 20.2g, preferably 15.2g of trisodium phosphate, placing them into a beaker, dissolving with water, transferring the resulting solution into a volumetric flask of 1 L, and then supplementing water into the volumetric flask to reach the mark;
   the buffer solution B is prepared according to the following steps: weighing 5.5 to 8.0 g, preferably 7.0 g of p-aminobenzene sulfonic acid, 60.5g to 83.0 g, preferably 71.6 g of disodium hydrogen phosphate, 5.0g to 7.0 g, preferably 6.2g of sodium dihydrogen phosphate, and 10.0g to 12.1g, preferably 11.8g of sodium citrate, placing them into a beaker, dissolving with water, transferring the resulting solution into a volumetric flask of 1 L, and then supplementing water into the volumetric flask to reach the mark;
   the ratio of the amount of the buffer solution A to the amount of the buffer solution B is 3: 4.

2. A continuous flow method for determining total alkaloids in tobacco or tobacco products, comprising generating online cyanogen chloride by using potassium rhodanide and sodium dichloroisocyanurate, reacting the cyanogen chloride with the total alkaloids (by nicotine) in the tobacco or the tobacco products, breaking the pyridine ring of the nicotine, reacting the resulting substance with p-aminobenzene sulfonic acid, and detecting the resulting product at 460nm using a colorimeter, the method comprising:

   controlling pH value of reaction system between 6.0 and 7.5 using a buffer solution A and a buffer solution B;
   wherein the buffer solution A is prepared according to the following steps: weighing 60.5 g to 83.0 g, preferably 71.6 g of disodium hydrogen phosphate and 10.1g to 20.2g, preferably 15.2g of trisodium phosphate, placing them into a beaker, dissolving with water, transferring the resulting solution into a volumetric flask of 1 L, and then supplementing water into the volumetric flask to reach the mark;
   the buffer solution B is prepared according to the following steps: weighing 5.5 to 8.0 g, preferably 7.0 g of p-aminobenzene sulfonic acid, 60.5g to 83.0 g, preferably 71.6 g of disodium hydrogen phosphate, 5.0g to 7.0 g, preferably 6.2g of sodium dihydrogen phosphate, and 10.0g to 12.1g, preferably 11.8g of sodium citrate, placing them into a beaker, dissolving with water, transferring the resulting solution into a volumetric flask of 1 L, and then supplementing water into the volumetric flask to reach the mark; and
   the ratio of the amount of the buffer solution A to the amount of the buffer solution B is 3: 4.

3. The method according to claim 2, **characterized in that** the method further comprising:
   preparing a sample according to the following steps: preparing a tobacco sample according to the principle of YC / T31, detecting the moisture content, extracting the tobacco sample with 5% acetic acid, and then filtering to obtain a filtrate.

4. The method according to any one of claims 2-3, **characterized in that** the method further comprising:
   preparing a standard solution according to the following steps: weighing nicotine or nicotine salts, dissolving the same with 5% acetic acid, formulating into at least five standard solutions, the nicotine content in the formulated standard solutions ranges from 0.5% to 7%;

5. The method according to any one of claims2-4, **characterized in that** the method further comprising:

   preparing a detoxification solution A according to the following steps: weighing 1g of citric acid, and 10g of ferrous sulfate, placing them into a beaker, dissolving with water, transferring the resulting solution into a volumetric flask of 1L, and then supplementing water into the volumetric flask to reach the mark;
   preparing a detoxification solution B according to the following steps: weighing 10g of sodium carbonate, placing it into a beaker, dissolving with water, transferring the resulting solution into a volumetric flask of 1L, and then supplementing water into the volumetric flask to reach the mark;

preparing a potassium rhodanide solution according to the following steps: weighing potassium rhodanide, placing it into a beaker, dissolving with water, transferring the resulting solution into a volumetric flask of 250mL, and then supplementing water into the volumetric flask to reach the mark, the concentration of the solution ranges from 0.09 to 0.21mol/L, preferably 0.12 mol/L;

preparing a sodium dichloroisocyanurate solution according to the following steps: weighing sodium dichloroisocyanurate, placing it into a beaker, dissolving with water, transferring the resulting solution into a volumetric flask of 250mL, and then supplementing water into the volumetric flask to reach the mark, the concentration of the solution ranges from 0.02 to 0.06mol/L, preferably 0.04 mol/L;

preferably, in the method, the ratio of the concentration of the potassium rhodanide solution and the concentration of the sodium dichloroisocyanurate solution is 2.5: 1 to 3.5: 1, preferably 3: 1.

6. The method according to any one of claims 2-5, **characterized in that** the method further comprising:

detecting by a continuous flow analyzer, then calculating the total alkaloids in the sample according to the following formula:

$$\text{total alkaloids\%} \quad = \frac{X \times V}{m \times (1-W) \times 1000} \times 100$$

in the formula,

X: observed value of the sample(s) in milligrams per milliliter (mg/mL);
V: volume of the added extract solution in milliliters (mL);
m: mass of the sample(s) in grams (g);
W: percentage of moisture in the sample(s), % (mass fraction);the total alkaloid in the above formula is based on dry basis.

7. The method according to any one of claims 2-6, **characterized in that** the operating parameters of the continuous flow analyzer in the method are: the ratio of the sampling time to the cleaning time is 1.5, the sampling frequency is 48 cups/hour, the gain is 18-60, and the light intensity is 2.85 volts.

**Patentansprüche**

1. Puffersystem zur Bestimmung der Gesamtalkaloide in Tabak oder Tabakerzeugnissen durch ein kontinuierliches Fließverfahren, umfassend eine Pufferlösung A und eine Pufferlösung B;

wobei die Pufferlösung A gemäß den folgenden Schritten hergestellt wird: Abwiegen von 60,5 g bis 83,0 g, vorzugsweise 71,6 g Dinatriumhydrogenphosphat und 10,1 g bis 20,2 g, vorzugsweise 15,2 g Trinatriumphosphat, Einfüllen in ein Becherglas, Auflösen mit Wasser, Überführen der resultierenden Lösung in einen Messkolben von 1 L und anschließendes Nachfüllen von Wasser in den Messkolben, um die Markierung zu erreichen;

die Pufferlösung B gemäß den folgenden Schritten hergestellt wird: Abwiegen von 5,5 bis 8,0 g, vorzugsweise 7,0 g p-Aminobenzolsulfonsäure, 60,5 g bis 83,0 g, vorzugsweise 71,6 g Dinatriumhydrogenphosphat, 5,0 g bis 7,0 g, vorzugsweise 6,2 g Natriumdihydrogenphosphat und 10,0 g bis 12,1 g, vorzugsweise 11,8 g Natriumcitrat, Einfüllen in ein Becherglas, Auflösen mit Wasser, Überführen der resultierenden Lösung in einen Messkolben von 1 L und anschließendes Nachfüllen von Wasser in den Messkolben, um die Markierung zu erreichen; und

das Verhältnis der Menge der Pufferlösung A zu der Menge der Pufferlösung B 3:4 beträgt.

2. Kontinuierliches Fließverfahren zur Bestimmung der Gesamtalkaloide in Tabak oder Tabakerzeugnissen, umfassend Erzeugen von Online-Chlorcyan unter Verwendung von Kaliumrhodanid und Natriumdichlorisocyanurat, Umsetzen des Chlorcyans mit den Gesamtalkaloiden (von Nikotin) in dem Tabak oder den Tabakerzeugnissen, Brechen des Pyridinrings des Nikotins, Umsetzen der resultierenden Substanz mit p-Aminobenzolsulfonsäure und Detektieren des resultierenden Produkts bei 460 nm unter Verwendung eines Colorimeters, wobei das Verfahren umfasst:

Regeln des pH-Wertes des Reaktionssystems zwischen 6,0 und 7,5 unter Verwendung einer Pufferlösung A und einer Pufferlösung B;

wobei die Pufferlösung A gemäß den folgenden Schritten hergestellt wird: Abwiegen von 60,5 g bis 83,0 g, vorzugsweise 71,6 g Dinatriumhydrogenphosphat und 10,1 g bis 20,2 g, vorzugsweise 15,2 g Trinatriumphos-

**EP 3 168 605 B1**

phat, Einfüllen in ein Becherglas, Auflösen mit Wasser, Überführen der resultierenden Lösung in einen Messkolben von 1 L und anschließendes Nachfüllen von Wasser in den Messkolben, um die Markierung zu erreichen; die Pufferlösung B gemäß den folgenden Schritten hergestellt wird: Abwiegen von 5,5 bis 8,0 g, vorzugsweise 7,0 g p-Aminobenzolsulfonsäure, 60,5 g bis 83,0 g, vorzugsweise 71,6 g Dinatriumhydrogenphosphat, 5,0 g bis 7,0 g, vorzugsweise 6,2 g Natriumdihydrogenphosphat und 10,0 g bis 12,1 g, vorzugsweise 11,8 g Natriumcitrat, Einfüllen in ein Becherglas, Auflösen mit Wasser, Überführen der resultierenden Lösung in einen Messkolben von 1 L und anschließendes Nachfüllen von Wasser in den Messkolben, um die Markierung zu erreichen; und

das Verhältnis der Menge der Pufferlösung A zu der Menge der Pufferlösung B 3:4 beträgt.

3. Verfahren nach Anspruch 2, **dadurch gekennzeichnet, dass** das Verfahren ferner umfasst:
Herstellen einer Probe gemäß der folgenden Schritte: Herstellen einer Tabakprobe nach dem YC/T31-Prinzip, Detektieren des Feuchtigkeitsgehalts, Extrahieren der Tabakprobe mit 5% Essigsäure und anschließendes Filtrieren, um ein Filtrat zu erhalten.

4. Verfahren nach einem der Ansprüche 2-3, **dadurch gekennzeichnet, dass** das Verfahren ferner umfasst:
Herstellen einer Standardlösung gemäß den folgenden Schritten: Abwiegen von Nikotin oder Nikotinsalzen, Auflösen derselben mit 5% Essigsäure, Formulieren in mindestens fünf Standardlösungen, wobei der Nikotingehalt in den formulierten Standardlösungen im Bereich von 0,5% bis 7% liegt.

5. Verfahren nach einem der Ansprüche 2-4, **dadurch gekennzeichnet, dass** das Verfahren ferner umfasst:

Herstellen einer Entgiftungslösung A gemäß den folgenden Schritten: Abwiegen von 1 g Zitronensäure und 10 g Eisensulfat, Einfüllen in ein Becherglas, Auflösen mit Wasser, Überführen der resultierenden Lösung in einen Messkolben von 1 L und anschließendes Nachfüllen von Wasser in den Messkolben, um die Markierung zu erreichen;
Herstellen einer Entgiftungslösung B gemäß den folgenden Schritten: Abwiegen von 10 g Natriumcarbonat, Einfüllen in ein Becherglas, Auflösen mit Wasser, Überführen der resultierenden Lösung in einen Messkolben von 1 L und anschließendes Nachfüllen von Wasser in den Messkolben, um die Markierung zu erreichen;
Herstellen einer Kaliumrhodanidlösung gemäß den folgenden Schritten: Abwiegen von Kaliumrhodanid, Einfüllen in ein Becherglas, Auflösen mit Wasser, Überführen der resultierenden Lösung in einen Messkolben von 250 ml und anschließendes Nachfüllen von Wasser in den Messkolben, um die Markierung zu erreichen, wobei die Konzentration der Lösung im Bereich von 0,09 bis 0,21 mol/L, vorzugsweise bei 0,12 mol/L, liegt;
Herstellen einer Natriumdichlorisocyanuratlösung gemäß den folgenden Schritten: Abwiegen von Natriumdichlorisocyanurat, Einfüllen in ein Becherglas, Auflösen mit Wasser, Überführen der resultierenden Lösung in einen Messkolben von 250 ml und anschließendes Nachfüllen von Wasser in den Messkolben, um die Markierung zu erreichen, wobei die Konzentration der Lösung im Bereich von 0,02 bis 0,06 mol/L, vorzugsweise bei 0,04 mol/L, liegt;
wobei vorzugsweise in dem Verfahren das Verhältnis der Konzentration der Kaliumrhodanidlösung und der Konzentration der Natriumdichlorisocyanuratlösung 2,5:1 bis 3,5:1, vorzugsweise 3:1, beträgt.

6. Verfahren nach einem der Ansprüche 2-5, **dadurch gekennzeichnet, dass** das Verfahren ferner umfasst:
Detektieren durch einen kontinuierlichen Durchflussanalysator und anschließendes Berechnen der Gesamtalkaloide in der Probe gemäß der folgenden Formel:

$$\text{Gesamtalkaloide \%} = \frac{X \times V}{m \times (1-W) \times 1000} \times 100$$

wobei in der Formel

X: beobachteter Wert der Probe(n) in Milligramm pro Milliliter (mg/mL);
V: Volumen der zugegebenen Extraktlösung in Millilitern (mL);
m: Masse der Probe(n) in Gramm (g);
W: prozentualer Feuchtigkeitsgehalt in der/den Probe(n), % (Massenanteil);
wobei das Gesamtalkaloid in der obigen Formel auf Trockenbasis basiert.

7. Verfahren nach einem der Ansprüche 2-6, **dadurch gekennzeichnet, dass** die Betriebsparameter des kontinuier-

23

lichen Durchflussanalysators in dem Verfahren wie folgt sind: das Verhältnis der Probenahmezeit zu der Reinigungszeit beträgt 1,5, die Probenahmefrequenz beträgt 48 Cups/Stunde, die Verstärkung beträgt 18-60 und die Lichtintensität beträgt 2,85 Volt.

## Revendications

1. Système tampon pour la détermination des alcaloïdes totaux dans le tabac ou les produits du tabac par un procédé à écoulement continu, comprenant une solution tampon A et une solution tampon B; dans laquelle la solution tampon A est préparée selon les étapes suivantes:

   peser 60,5 g à 83,0 g, de préférence 71,6 g d'hydrogénophosphate disodique et 10,1 g à 20,2 g, de préférence 15,2 g de trisodiumphosphate, les placer dans un bécher, les dissoudre dans l'eau, transférer la solution obtenue dans un ballon jaugé de 1 L, puis compléter avec de l'eau pour atteindre le repère;
   la solution tampon B est préparée selon les étapes suivantes:

   peser 5,5 à 8,0 g, de préférence 7,0 g d'acide p-aminobenzène sulfonique, 60,5 g à 83,0 g, de préférence 71,6 g d'hydrogénophosphate disodique, 5,0 g à 7,0 g, de préférence 6,2 g de dihydrogène phosphate de sodium et 10,0g à 12.1g, de préférence 11,8 g de citrate de sodium, les placer dans un bécher, les dissoudre avec de l'eau, transférer la solution obtenue dans un ballon jaugé de 1 L, puis ajouter de l'eau dans le ballon jaugé pour atteindre le repère;
   le rapport entre la quantité de la solution tampon A et la quantité de la solution tampon B est de 3:4. 4.

2. Procédé à écoulement continu pour déterminer les alcaloïdes totaux dans le tabac ou les produits du tabac, comprenant la génération de chlorure de cyanogène en ligne en utilisant du rhodanure de potassium et du dichloroisocyanurate de sodium, la réaction du chlorure de cyanogène avec les alcaloïdes totaux (par nicotine) dans le tabac ou les produits du tabac, la rupture du noyau pyridine de la nicotine, la réaction de la substance obtenue avec l'acide p-aminobenzènesulfonique et la détection du produit obtenu à 460 nm en utilisant un colorimètre, le procédé comprenant les étapes suivantes:

   réguler le pH du système réactionnel entre 6,0 et 7,5 à l'aide d'une solution tampon A et d'une solution tampon B; dans laquelle la solution tampon A est préparée selon les étapes suivantes:

   peser 60,5 g à 83,0 g, de préférence 71,6 g d'hydrogénophosphate disodique et 10,1 g à 20,2 g, de préférence 15,2 g de trisodiumphosphate, les placer dans un bécher, les dissoudre dans l'eau, transférer la solution obtenue dans un ballon jaugé de 1 L, puis ajouter de l'eau dans le ballon jaugé pour atteindre le repère;
   la solution tampon B est préparée selon les étapes suivantes:

   peser 5,5 à 8,0 g, de préférence 7,0 g d'acide p-aminobenzène sulfonique, 60,5 g à 83,0 g, de préférence 71,6 g d'hydrogénophosphate disodique, 5,0 g à 7,0 g, de préférence 6,2 g de dihydrogène phosphate de sodium et 10,0g à 12.1g, de préférence 11,8 g de citrate de sodium, les placer dans un bécher, les dissoudre avec de l'eau, transférer la solution obtenue dans un ballon jaugé de 1 L, puis ajouter de l'eau dans le ballon jaugé pour atteindre le repère; et
   le rapport entre la quantité de la solution tampon A et la quantité de la solution tampon B est de 3:4.

3. Procédé selon la revendication 2, **caractérisé en ce que** le procédé comprend en outre:
   la préparation d'un échantillon selon les étapes suivantes:
   la préparation d'un échantillon de tabac selon le principe YC/T31, la détection de la teneur en humidité, l'extraction de l'échantillon de tabac avec de l'acide acétique à 5%, puis la filtration pour obtenir un filtrat.

4. Procédé selon l'une quelconque des revendications 2 à 3, **caractérisé en ce que** le procédé comprend en outre:
   la préparation d'une solution étalon selon les étapes suivantes:
   la pesée de la nicotine ou des sels de nicotine, la dissolution avec 5% d'acide acétique, la formulation en au moins cinq solutions étalons, la teneur en nicotine dans les solutions étalons formulées varie de 0,5 à 7 %.

5. Procédé selon l'une quelconque des revendications 2 à 4, **caractérisé en ce que** le procédé comprend en outre:
   la préparation d'une solution de détoxification A selon les étapes suivantes:

peser 1g d'acide citrique et 10g de sulfate ferreux, les placer dans un bécher, les dissoudre avec de l'eau, transférer la solution obtenue dans un ballon jaugé de 1L, puis ajouter de l'eau dans le ballon jaugé pour atteindre le repère;

la préparation une solution de détoxification B selon les étapes suivantes:

peser 10g de carbonate de sodium, les placer dans un bécher, les dissoudre avec de l'eau, transférer la solution obtenue dans un ballon jaugé de 1L, puis ajouter de l'eau dans le ballon jaugé pour atteindre le repère;

préparer une solution de rhodanure de potassium selon les étapes suivantes:

peser du rhodanure de potassium, les placer dans un bécher, les dissoudre avec de l'eau, transférer la solution obtenue dans un ballon jaugé de 250mL, puis ajouter de l'eau dans le ballon jaugé pour atteindre le repère, la concentration de la solution varie de 0,09 à 0,21 mole/L, de préférence 0,12 mole/L; préparer une solution de dichloroisocyanurate de sodium selon les étapes suivantes: peser du dichloroisocyanurate de sodium, les placer dans un bécher, les dissoudre avec de l'eau, transférer la solution obtenue dans un ballon jaugé de 250mL, puis ajouter de l'eau dans le ballon jaugé pour atteindre le repère, la concentration de la solution varie de 0,02 à 0,06 mole/L, de préférence 0,04 mole/L; de préférence, dans le procédé, le rapport de la concentration de la solution de rhodanure de potassium et de la concentration de la solution de dichloroisocyanurate de sodium est de 2,5:1 à 3,5:1, de préférence 3:1.

6. Procédé selon l'une quelconque des revendications 2 à 5, **caractérisé en ce que** le procédé comprend en outre: la détection par un analyseur à flux continu, puis le calcul des alcaloïdes totaux dans l'échantillon selon la formule suivante:

$$\text{total d'alcaloïdes en \%} = \frac{\frac{X \times V}{m \times (1-W) \times 1000} \times 100}{}$$

dans la formule,

X: valeur observée de l'échantillon ou des échantillons en milligrammes par millilitre (mg/mL);
V: volume de la solution d'extrait ajoutée en millilitres (mL);
m: masse de l'échantillon ou des échantillons en grammes (g);
W: pourcentage d'humidité dans l'échantillon ou les échantillons, % (fraction massique);
l'alcaloïde total dans la formule ci-dessus est fondé sur une base sèche.

7. Procédé selon l'une quelconque des revendications 2 à 6, **caractérisé en ce que** les paramètres de fonctionnement de l'analyseur à flux continu dans le procédé sont les suivants: le rapport du temps d'échantillonnage au temps de nettoyage est de 1,5, la fréquence d'échantillonnage est de 48 tasses/heure, le gain est de 18-60 et l'intensité lumineuse est de 2,85 volts.

Figure 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 104132895

- CN 104132937 A